Europäisches Patentamt

European Patent Office

Office européen des brevets

(11) Publication number: **0 686 696 A1**

## EUROPEAN PATENT APPLICATION

(21) Application number: 95201535.2

(22) Date of filing: 09.06.95

(51) Int. Cl.⁶: **C12N 15/13**, C07K 16/08, C07K 1/22, G01N 33/577

The applicant has subsequently filed a sequence listing and declared, that it includes no new matter.

(30) Priority: 09.06.94 CU 7394

(43) Date of publication of application:
13.12.95 Bulletin 95/50

(84) Designated Contracting States:
AT BE CH DE DK ES FR GB GR IE IT LI LU MC NL PT SE

(71) Applicant: **CENTRO DE INGENIERIA GENETICA Y BIOTECNOLOGIA**
**31 Street, '/156 & 190,**
**Cubanacan Playa**
**Havana (CU)**

(72) Inventor: **Ayala Avila, Marta**
**Calle 186 No 3117 entre 31 y 33,**
**Apto 3D,**
**Playa**
**C. de la Habana (CU)**
Inventor: **Gavilondo Cowley, Jorge Victor**
**Calle G No. 460 entre 19 y 21,**

**Plaza de la Revolucion,**
**C. de la Habana (CU)**
Inventor: **Fernandez de Cossio Dorta-Duque, Maria Elena**
**Ave. 31 no. 18207 entre 182 y 184,**
**Apto 27 Piso 6**
**Playa,**
**C. de la Habana (CU)**
Inventor: **Canaan-Haden Frias, Leonrado Miguel**
**Calle 186 No. 3117 entre 31 y 33,**
**Apto 3D**
**Playa,**
**C. de la Habana (CU)**
Inventor: **del Carmen Dominguez Horta, Maria**
**Calle 13 no. 2016 entre 20 y 22,**
**Matanzas**
**Matanzas (CU)**

(74) Representative: **Smulders, Theodorus A.H.J., Ir. et al**
**Vereenigde Octrooibureaux**
**Nieuwe Parklaan 97**
**NL-2587 BN 's-Gravenhage (NL)**

(54) **Recombinant single chain Fv antibody fragment and its use in the immunopurification of the recombinant Hepatitis B Virus Surface Antigen**

(57) A recombinant single chain Fv (scFv) antibody fragment specific for the Hepatitis B virus surface antigen (HBsAg). Production of the scFv fragment in *Escherichia coli* using recombinant DNA technology. The scFv can substitute the mouse monoclonal antibody (MAb) CB-Hep.1 that is used in one of the purification steps of a known production process of such antigen. The scFv antibody fragment is developed using recombinant DNA technology, by cloning and modifying the genes that code for the heavy and light chain variable regions of MAb CB-Hep.1. The scFv fragment is specific for the HBsAg, has smaller size than the MAb, and is produced from bacterial cultures *(E. coli)*. The scFv is purified from material harvested from these cultures and immobilized on chromatographic supports for its use as immunoabsorbent in the purification of the recombinant HBsAg that is employed as active raw material of a vaccine preparation. Due to its specificity for the HBsAg, the fragment can be employed also in immunoassays in which the HBsAg is recognized or detected. Part of the protein sequence of the heavy chain variable region of MAb CB-Hep.1 may be used to construct fusion proteins for expression in *E. coli* that will associate with the bacterial inner membrane, facilitating in this way their purification process.

Figure 1.

FIELD OF THE INVENTION:

The present invention is related to the field of biotechnology and in particular with the development of a single chain Fv (scFv) antibody fragment using recombinant DNA technology and its use in the purification process of a recombinant Hepatitis B virus surface antigen (HBsAg) that constitutes the active raw material of a vaccine.

BACKGROUND OF THE INVENTION:

The commercially available recombinant vaccines against Hepatitis B are essentially based on the production of recombinant HBsAg in genetically manipulated yeast.

The yeast *Saccharomyces cerevisiae* has been widely employed for the large scale production of recombinant HBsAg (McAleer, W.J. et al. 1984. Nature 307:178; Harford, N. et al. 1987. Postgraduate Medical Journal 63 suppl 2:65; Bitter, G.A. et al. 1988. Journal of Medical Virology 25:123). The antigen is produced intracellularly and is extracted by different cell disruption methods and purified according to different physico-chemical procedures that have allowed purity levels higher that 97% and a product with a similar antigenic behaviour to the plasma-derived antigen, as evidenced in animals and humans (Hauser, P. et al. 1987. Postgraduate Medical Journal, 63 suppl 2:83).

In the European Patent Application No. 480 525 a purification method for a recombinant HBsAg obtained in the yeast *Pichia pastoris* is described. The method involves an immunoaffinity chromatography step with the specific mouse monoclonal antibody (MAb) CB-Hep.1. The purification process confers to the HBsAg immunogenicity characteristics that make it superior as immunogen when compared to the vaccine "Engerix B", obtained by recombinant DNA technology in *Saccharomyces cerevisiae,* and marketed by the company SmithKline Beecham Biologicals.

The use of MAb as ligand in the preparation of affinity supports for the development of purification processes of natural and recombinant molecules is very well known (Mattiasson B. 1988. Methods in Enzymology, Vol. 137; Avrameas, S. and T. Ternynck. 1969. Immunochemistry 6: 53-66; Bethell, G. S. et. al. 1979. J. Biol. Chem. 254: 2572-2574; Chase, H. A. 1984. Chem. Eng. Sci. 39: 1099-1125; Sada, E. 1986. et. al Biotechnol. Bioeng. 28: 1497-1502).

One of the most important steps in the purification process of the HBsAg from *Pichia pastoris,* object of the European Patent Application 480525, is in fact the operation of immunopurification of the antigen, using the MAb CB-Hep.1, that is supposed to contribute to a selection of very antigen particles of high immunogenicity. The hybridoma which secretes the MAb CB-Hep.1 (Fontirrochi et al. 1993. Biotecnología Aplicada 10, 1:24-30) was obtained from a BALB/c mouse immunized with recombinant antigen, after the fusion of its spleen cells with the mouse myeloma $Sp^2/0$- Ag14. When coupled to Sepharose, the MAb efficiently purifies the antigen present in preparations of the recombinant yeast.

Production of MAb for such use can be developed both "in vitro", employing bioreactors and harvesting the hybridoma culture supernatant (Handa-Corrigan, 1988. A. Bio/Technology 6:784-786), or "in vivo", with mice that are inoculated intraperitoneally with hybridomas, and develop ascitic tumors with abundant fluid rich in MAb (Campbell, A. (Edit). 1984. Monoclonal Antibody Technology. Vol.13, Laboratory. Techniques in Biochemistry and Molecular Biology, Elsevier Pub. Co.). In any case, the international regulations for the quality and safety of use of MAbs are very strict (Office of Biologics Research and Review, Center for Drugs and Biologics, FDA. Points to Consider in the Manufacture and Testing of Monoclonal Antibody Products for Human Use. Memorandum, 1993), with the concomitant increase in the costs of production and validations to assure and show the absence of adventitious agents that could represent a potential biohazard when the MAbs are to be used directly in humans or in the production process of pharmaceuticals and vaccines.

The development of recombinant DNA technology has allowed the production of Fab, Fv, and scFv antibody fragments in microorganisms (Plückthun, A. 1991. Bio/Technology 9:545-551; Whitlow, M. and D. Filpula, 1991. In METHODS: A Companion to Methods in Enzymology. Vol 2. No.2. pp 97-105).

The US patent No. 4,946,778 of Genex Corporation, acquired afterwards by Enzon Incorporated, describes the technology involved in the production of scFv fragments (also denominated SCA: single-chain antigen-binding proteins) with a substantially similar behaviour in terms of specificity and affinity, to whole antibodies.

The US patent No. 4,816,567 of Genentech Incorporated, describes the preparation of recombinant immunoglobulins, both of chimeric mouse-human antibodies, and chimeric Fab fragments.

In the Proceedings of the 1st. International Conference "Antibody Engineering: New Technology and Application Implications", developed in San Diego, California, USA, in 1990 (organized by IBC USA Conferences Inc.), and of the 4th. International Conference on Antibody Engineering, celebrated in Coronado, California, USA, in 1993 (same organizers), Cytogen Corporation reported a synthetic peptide obtained from the information contained in the aminoacid sequence of the CDR3 complementarity determining region of the heavy chain variable domain of a mouse MAb, specific for human platelet fibrinogen receptor. The synthetic peptide maintains the antigen recognition properties of the original antibody.

Recently, M.J. Berry et al. (Journal of Chromatography, 587: 161-169, 1991) report the immobilization in porous silica of an Fv antibody fragment produced by recombinant DNA technology, and its use in the purification of hen egg lysozyme, in comparison with the whole antibody. The authors point out the reasons why antibody fragments are convenient for immunoaffinity chromatography, among them, their microbial production in *E. coli*, which lowers costs with respect to whole antibodies produced in mammalian cell culture. Also, fragments have a higher antigen capacity, based on mass to mass ratio. Finally, fragments are small enough as to be immobilized inside the pores of the silica matrix without a significant reduction in pore size.

M.J. Berry and J.J. Pierce (Journal of Chromatography, 629:161-168, 1993) also demonstrated the possibility of using both Fv and scFv fragments, immobilized on cyanogen bromide activated Sepharose, for the immunopurification of hen egg lysozyme. In their study they show the stability of these immunosorbents after 100 cycles of purification, regarding their capacity to trap antigen.

More recently T. M. Spitznagel and D. S. Clark published a paper related to the study of the surface density and orientation of immobilized antibodies and antibody fragments. They concluded that Fv have better antigen binding capacity than Fab or whole antibodies. In this way they show that the removal of unnecessary protein domains (i.e. constant regions in antibodies) can be beneficial for the improvement of the total capacity of immunosorbents (Bio/Technology, 11:825-828, 1993).

## SUMMARY OF THE INVENTION:

The present invention constitutes an alternative to the use of the MAb CB-Hep.1 (European Patent Office Application No. 480525) in the immunoaffinity chromatography step of the purification process of the vaccine antigen. With the scFv constructed from the genes that code for the variable regions of MAb CB-Hep.1, the use of eukaryotic cells (hybridomas) in culture and laboratory animals (BALB/c mice) as sources for MAb is prevented. The preparation and validation steps that the MAb and the affinity supports constructed with it require, in order to comply with established international guidelines that guarantee the absence of adventitious agents, associated wih hybridomas, the serum used in culture techniques, or laboratory mice (Office of Biologics Research and Review, Center for Drugs and Biologics, FDA. Points to Consider in the Manufacture and Testing of Monoclonal Antibody Products for Human Use. Memorandum, 1993), are substantially reduced and simplified when the scFv is used in place of the whole antibody. The same happens regarding the validation procedures of the final preparation of vaccine antigen purified by immunoaffinity (Office of Biologics Research and Review, Center for Drugs and Biologics, FDA. Points to Consider in the Production and Testing of New Drugs and Biologicals Produced by Recombinant DNA Technology. Draft, 1985).

Due to the fact that it is produced in *E. coli*, the scFv substantially reduces and simplifies the regulatory aspects related to the use of MAb, with a consequent lowering of costs, and increases the potential production capacity of the vaccine antigen, based on the possibilities of fermentative scaling-up of *E. coli*. The fragment obtained by the present invention and its use in the purification of the HBsAg antigen constitute objects of the present invention.

An unexpected result derived from the present technical solution was the finding of the property of the scFv fragment of associating to the *E. coli* inner membrane, instead of being transported to the bacterial periplasm. This property, associated to the particular aminoacid sequence of the heavy chain variable region of MAb CB-Hep.1, can be transfered to other fusion proteins to be expressed in *E. coli* with the purpose of facilitating purification. This aspect constitutes another object of the present invention.

## BRIEF DESCRIPTION OF THE DRAWINGS:

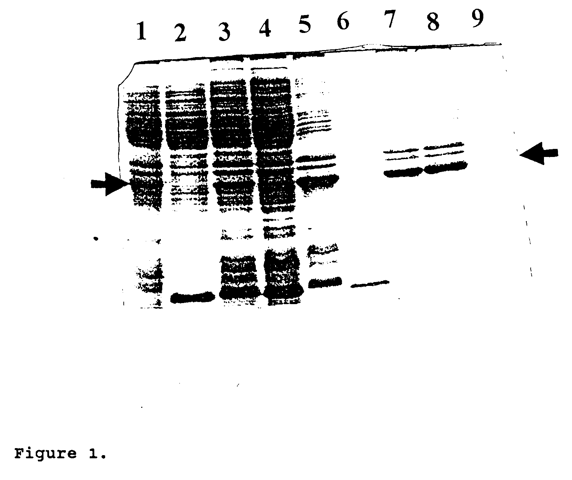

Figure 1: The analysis of biomass samples in 15% SDS-polyacrylamide gels indicated that under these conditions, a 32-33 kDa new protein is expressed, insoluble, and associated with cell structures. Its expression level in MM294 is around 15-20% of total bacterial protein.

DETAILED DISCLOSURE OF THE INVENTION:

For the construction of the scFv antibody fragment specific for the HBsAg, first, the total RNA of the hybridoma cells secreting MAb CB-Hep.1 was extracted. This RNA was used as template for first strand DNA synthesis (cDNA), and with the latter the specific amplification reactions for the genes encoding the heavy (VH) and light (VL) chain variable regions were done using the Polymerase Chain Reaction technique (PCR, Oste, C. 1988. BioTechniques 6:162-167) and a set of synthetic oligonucleotides, as described previously (Ayala, M. et al. 1992. BioTechniques 13:790-799).

The amplified VH and VL regions were subcloned and sequenced to obtain the consensus nucleotide sequence. From such sequences, new synthetic oligonucleotides were designed in order to construct the scFv, with the order VH-Linker-VL. The employed linker structure was that reported by Chaudhary et al. (Proc. Natl. Acad. Sci. USA 87: 1066-1070, 1990). The scFv fragment, obtained using PCR, and after digestion with convenient restriction enzymes, was ligated to the expression vector pPACIB.1 (J. Gavilondo et al. Proceedings of the 4th. International Conference "Antibody Engineering", December 1993, Coronado, USA), previously digested with the same set of enzymes. The ligated material was used to transform E. coli MC1061 competent cells, and the resulting colonies that grew on selective semisolid medium were checked for the presence of the cloned gene, using restriction digestion.

The selected plasmid, denominated pPACIB.1/scFv anti HBsAg, was then used to transform different E. coli strains (MM294, coliB, W3110, BMH 71-18) for the expression study. Expression of a new intracellular insoluble protein of molecular size ca. 32-33 kDa was identified in recombinant MM294 and coliB cells using electrophoresis in SDS polyacrylamide gels (SDS-PAGE). Western blot studies with specific antisera showed that the protein was the anti-HBsAg scFv.

To evaluate the biological activity of this new protein, the transformed bacterial strain MM294 was grown in 300 ml shaker flasks and the scFv extracted from the membrane fraction, using 6 M urea. The solubilized protein was extensively dialyzed against phosphate buffer pH 8.0, and tested in an ELISA where polystyrene plates were coated with the recombinant HBsAg, and the fragment-antigen reaction evidenced using specific anti-Fab of CB-Hep.1 horseradish peroxidase conjugated antibodies.

Once the specific recognition of the scFv for the HBsAg was shown in ELISA, the fragment was purified from the solubilized and dialyzed protein, as mentioned above. Immobilized metal affinity chromatography (Sepharose-IDA) was used, taking advantage of the characteristic given to the scFv by the expression plasmid pPACIB.1 of producing the protein with a 6-histidine domain (a domain of six histidines) in the amino-terminus.

The purified scFv was tested for its ability to bind recombinant HBsAg in solution. In these experiments different concentrations of scFv and recombinant HBsAg were mixed, the samples assayed in the aforementioned ELISA system, and signal inhibition recorded. It was shown that the preincubation of scFv and recombinant HBsAg will reduce the binding of the scFv to the antigen coated to the solid phase.

Finally, the anti-HBsAg was immobilized to cyanogen bromide activated Sepharose CL-4B and the support tested for absorption and desorption of both pure HBsAg, and a crude recombinant yeast HBsAg extract. These tests evidenced the capacity of the anti-HBsAg scFv to purify the antigen.

EXAMPLES: These examples intend to illustrate the invention, but not to limit its scope.

Example 1.-

Amplification of the genes that encode the heavy and light chain variable regions (VH and VL, respectively) of the MAb CB-Hep.1, using the polymerase chain reaction (PCR). Cloning and base sequencing.-

Procedure (a). RNA isolation.-

The isolation of total RNA from hybridoma cells secreting MAb CB-Hep.1 was done by the method suggested by Gough et al. (Anal. Biochem. 173:93-95, 1988). Briefly 5 x $10^6$ culture cells were harvested by centrifugation, and washed twice with phosphate buffered saline solution. The cells were suspended in 200 ul of cold solution A (10 mM Tris/HCl, 150 mM NaCl, 1.5 mM MgCl$_2$, 0.65% NP-40, pH 7.5), using a vortex mixer, the solution centrifuged at 8000 rpm for 5 min, transfered to a reaction vial with 200 ul of solution B (7 M urea, 1% SDS, 30 mM NaCl, 10 mM EDTA, 10 mM Tris/HCl pH 7.5), and gently mixed. Phenol : chloroform (1:1) was added, and submitted to vortex mixing for one minute. After a centrifugation at 12,000 rpm for 5 minutes, the aqueous phase was tranfered to a fresh reaction tube and the RNA precipitated with cold absolute EtOH and salts.

5

Procedure (b). cDNA synthesis.-

The RNA was used as template for the synthesis of first-strand complementary DNA (cDNA), using different deoxynucleotides, oligo dT as primer and reverse transcriptase (Gavilondo J.V. et. al. 1990. Hybridoma 9:407-417).

Procedure (c). PCR amplification.-

PCR was used for the specific amplification of VH and VL encoding genes of CB-Hep.1, from leader (signal peptide) to CH1 or Ck. Synthetic oligonucleotides were designed according to available databases on immunoglobulin variable region base sequences. PCR conditions were as described elsewhere (Gavilondo J.V. et. al. 1990. Hybridoma 9:407-417).

---------------------------------------------------------------------

Table I .- Synthetic oligonucleotides used for the specific PCR amplification of the genes coding for the VH and VL regions.


Heavy Chain Variable Region.

Oligo No.1

5' End: EcoRV-VH signal sequence.

5'...GCGGGATATCATG(AG)AA(CG)A(CG)CTGGGT(CT)(AT)T(CT)CTCT...3'


Oligo No.2

5' End: EcoRV-VH signal sequence.

5'...GCGGGATATCATG(AG)A(CG)TT(CG)(TG)GG(CT)T(AC)A(AG)CT(TG)G (AG)TT...3'


Oligo No.3

3' End: SalI-CH1 (IgG1, 2 and 3 from mouse).

5'...CCCGTCGACA(TC)CTCACACACAGG(AG)CCAGTGGATAGAC...3'


Light Chain Variable Region

Oligo No. 4

5' End: EcoRV-VL signal sequence

5'...GGGATATCATGGA(GA)(AT)CACA(GT)(AT)C(CT)CAGGTCTT(TC)(AG)TAT (TA)(CT)ACTG...3'


Oligo No.5

3' End: SalI-Ck mouse.

5'...CCGTCGACACTGGATGGTGGGAAGATGGA...3'


Note: Some primers were produced with degenerate positions, which appear in brackets.

---------------------------------------------------------------------

The amplified sequences were purified from low melting point agarose using phenol extraction, and digested with EcoRV and SalI for the cloning in sequencing vectors (Molecular Cloning, A Laboratory Manual, second edition, 1989, by Sambrook, Fritsch and Maniatis).

Procedure (d). Cloning of amplified VH and VL regions in plasmid Bluescript (pBS) II ks +/- for sequencing.-

The pBS was digested with the aforementioned restriction enzymes, and vector and PCR inserts ligated in two separate reactions: (1) pBS + VH and (2) pBS + VL. Ligation products were used for the transformation of competent *E. coli* cells (strain MC1061), that were then plated on semisolid selective medium and grown at 37°C (Molecular Cloning, A laboratory Manual, second edition, 1989, by Sambrook, Fritsch and Maniatis).

The recombinant vectors were selected after the purification of the plasmids from a number of bacterial colonies, and their digestion by restriction enzymes to check for the expected ligation product (Molecular Cloning, A Laboratory Manual, second edition, 1989, by Sambrook, Fritsch and Maniatis), that is, two bands corresponding to pBS (approx. 2.7 kb) and to the inserted variable domain (aprox. 500 pb). At least five clones for each domain (VH and VL) were selected for sequencing.

Procedure (e). Nucleotide sequence of the genes that encode for the VH and VL regions of MAb CB-Hep.1.-

The sequencing was done on the denatured chains (treatment with NaOH 10N) of the plasmid double-helixes according to the Sanger method (Molecular Cloning, A Laboratory Manual, second edition, 1989, by Sambrook, Fritsch and Maniatis), using universal primers (that hybridize outside from the multiple cloning region of the plasmid) and that allow the sequencing of the cloned gene in both directions.

The availability of databases with immunoglobulin variable region sequences, reported until 1991 (Kabat et al. (Kabat, E.A., T.T. Wu, H.M. Peery, K.S. Gottesman, C. Foeller 1991. Sequences of proteins of immunological interest. Vol. 11, 5th. Edition. U.S. Dept. Health and Human Services. NIH Publication No. 91-3242), allows the assignment of any new sequenced variable domain within one of the several defined subgroups. The VH region was assigned to subgroup IIIc, and VI to subgroup I.

VH Base (lowercase) and Aminoacid (uppercase) Sequences. Mab CB-Hep.1: (Sequence Identification No. 1)

```
atgaaatggagctgggttttttctcttaaaaggtgtccagtgt----------leader
  M   K   W   S   W   V   F   L   L   K   G   V   Q   C
-14                                             -1


gaggtgaagctggatgaaactggaggaggcttggtgcaacctgggaggcccatg
  E   V   K   L   D   E   T   G   G   G   L   V   Q   P   G   R   P   M
+1


aaactctcctgtgttgcctctggattcactttttagt---------------FR1
  K   L   S   C   V   A   S   G   F   T   F   S
                                          30


gacttctggatgaac-------------------------------------CDR1
  E   F   W   M   N
                  35


tgggtccgccagtctccggagaaaggactggagtgggtagca----------FR2
  W   V   R   Q   S   P   E   K   G   L   E   W   V   A
                                              49


caaatcagagacaaacctgataattatgcaatatattattcagaatctgtgaaa
  Q   I   R   D   K   P   D   N   Y   A   I   Y   Y   S   E   S   V   K
      52  a   b   c


ggc-------------------------------------------------CDR2
  G
65


agattcaccatctcaagagatgattccagaagtagtgtcttcctgcaaatgaac
  R   F   T   I   S   R   D   D   S   R   S   S   V   F   L   Q   M   N
                                                      82  a


agtttaagacctgaagatacgggtatctattactgtacggcc----------FR3
  S   L   R   P   E   D   T   G   I   Y   Y   C   T   A
  b   c                                       94


ggttttgactat---------------------------------------CDR3
  G   F   D   Y
  95          102


tggggccaaggcaccactctcacagtctcctca------------------FR4
  W   G   Q   G   T   T   L   T   V   S   S
103                                       113
```

VL Base (lowercase) and Aminoacid (uppercase) Sequences. MAb CB-Hep.1: (Sequence Identification No. 2)

```
atggattcacaggcccaggttcttatgttactgctgctatgggtatctggtacc
 M   D   S   Q   A   Q   V   L   M   L   L   L   W   V   S   G   T
-20

tgtggg---------------------------------------------leader
 C   G
    -1

gacattgtgatgtcacagtctccatcctccctagctgtgtcagttggagagaag
 D   I   V   M   S   Q   S   P   S   S   L   A   V   S   V   G   E   K
+1

gttgctttgagctgc--------------------------------------FR1
 V   A   L   S   C
                23

aagtccagtcagagtcttttatatcttaacaatcacaagaactacttggcc--CDR1
 K   S   S   Q   S   L   L   Y   L   N   N   H   K   N   Y   L   A
        27  a   b   c   d   e   f                           34

tggttccagcagaaacctgggcagtctcctaaactgctgatttac--------FR2
 W   F   Q   Q   K   P   G   Q   S   P   K   L   L   I   Y
                                                49

tgggcatccactagggattct-------------------------------CDR2
 W   A   S   T   R   D   S
                    56

ggggtccctgatcgcttcacaggcagtggatctgggacagatttcactctcatg
 G   V   P   D   R   F   T   G   S   G   S   G   T   D   F   T   L   M

atcagcagtgtgaaggctgaagacctggcagtttattactgt------------FR3
 I   S   S   V   K   A   E   D   L   A   V   Y   Y   C
                                            88

caacaatattataattatccgtacacg-------------------------CDR3
 Q   Q   Y   Y   N   Y   P   Y   T
                        97

ttcggagggggggaccaagctggaaataaaa---------------------FR4
 F   G   G   G   T   K   L   E   I   K
                            107
```

Example 2.-

Construction of a scFv antibody fragment (VH-linker-VL) starting from the VH and VL domains; cloning in an *E. coli* expression vector.

Pocedure (a). Construction of the scFv.-

The strategy involved a first PCR amplification to modify the sequenced VH and VL regions, including extra bases coding for half the linker (Chaudhary et al. 1990. Proc. Natl. Acad. Sci. USA 87:1066-1070) and restriction sites that will allow the ligation of the modified VH and VL, and the cloning of the scFv gene insert in an expression vector. Table II describes the synthetic oligonucleotides used for the PCR amplification, designed on the basis of the exact sequence of VH and VL.

```
------------------------------------------------------------

Table II. Synthetic oligonucleotides used in the first PCR

reaction for the construction of the scFv gene.-


Heavy Chain Variable Region


5' End:

Oligo No.5: EcoRI-FR1-VH.-

5'...GG/GAATTC/TGAGGTGAAGCTGGATGAAACTGG...3'


3' End:

Oligo No.6: BamHI-linker-FR4-VH:

5'..CCC/GGATCC/AGATCCTGAGGATTTACCCTCTGAGGAGACGGTGACCGTGGTGCC..3'



Light Chain Variable Region


5' End:

Oligo No.7: BamHI-linker-FR1-Vk

5'...GGG/GGATCC/GAATCCAAAGTCGACGACATTGAGCTCACCCAGTCT...3'


3' End:

Oligo No.8: EcoRV-FR4-Mouse Vk

5'...CC/GATATC/CTATTATTTTATTTCCAGCTTGGTCC...3'

------------------------------------------------------------
```

After amplification, the VH and VL modified regions were purified, digested with BamHI and ligated in equivalent proportions using T4 DNA ligase (Molecular Cloning, A Laboratory Manual, second edition, 1989, by Sambrook, Fritsch and Maniatis). The result of this ligation (a gene fragment of around 720 bp) was then submitted to a second PCR amplification with oligonucleotides 5 and 8 (Table II), in order to enrich the scFv material. The scFv was then purified, digested with EcoRI and EcoRV, and ligated to the pPACIB.1 vector, previously submitted to digestion with the same restriction enzymes. pPACIB.1 is a plasmid designed for periplasmic expression of heterologous proteins in *E. coli*. The plasmid has several regulatory sequences: promotor (tryptophan), an ompA signal peptide encoding region for protein secretion, and a 6-histidine encoding domain that will appear N-terminal to the mature expressed protein for purification using immobilized metal ion affinity chromatography (Gavilondo, J.V. et al. Proceedings of the IV Annual Conference on Antibody Engineering. IBC Conferences Inc. Coronado, California, USA. December 8-10, 1993).

The product of the ligation of vector and scFv gene insert was used to transform MC1061 *E. coli* competent cells, that were plated in semisolid selective medium and grown at 37°C. For the selection of recombinant vectors, the bacterial colonies were inoculated in liquid medium and submitted to a process for the extraction of the plasmid DNA (Molecular Cloning, A Laboratory Manual, second edition, 1989, by Sambrook, Fritsch and Maniatis). The plasmid DNA was then digested with the endonucleases EcoRI and EcoRV, and then applied to an agarose gel and visualized with UV light. The recombinant clones were selected among those exhibiting two bands as digestion pattern, one corresponding to the linearized pPACIB.1 (approx. 2.9 kb), and the second to the scFv (approx. 720 bp).

Procedure (b). Expression of the scFv in *E. coli.−*

Four *E. coli* strains (BMH71-18, coliB, W3110 and MM294) were transformed for expression studies using two recombinant plasmids selected in Procedure (a). Basically, the recombinant bacteria were grown in liquid medium (LB), with ampicillin, overnight at 37°C. Fresh cultures with LB and ampicillin were started, and incubated for 3 hours at 37°C. Protein expression was induced by adding beta-indoleacrylic acid to the culture. The analysis of biomass samples in 15% SDS-polyacrylamide gels indicated that under these conditions, a 32-33 kDa new protein is expressed, insoluble, and associated with cell structures. Its expression level in MM294 is around 15-20% of total bacterial protein (Figure 1).

It was shown by Western blot (Molecular Cloning, A Laboratory Manual, second edition, 1989, by Sambrook, Fritsch and Maniatis) using rabbit polyclonal antibodies raised against the Fab fragment of CB-Hep.1 (papain digestion), adsorbed against *E. coli* proteins to prevent background, and immunopurified with the recombinant antigen, that this protein was the anti-HBsAg scFv. The scFv protein could not be detected in periplasm neither by Western blot or a specific ELISA (see below).

Example 3.-

Production of scFv from bacterial cultures, renaturing and assays for specificity with the antigen.

Procedure (a). Extraction and renaturing of the anti-HBsAg scFv.-

After a process of extraction of the scFv protein from recombinant bacteria, involving ultrasound disrupture, and separation of soluble and insoluble cell fractions, combined with SDS-polyacrylamide gel electrophoresis, transfer onto nitrocellulose and Western blot, it was found that the scFv protein remains associated to the insoluble cell fraction. Transmission electron microscopy studies indicated the absence of inclusion bodies, suggesting that the scfv protein could remain associated to the bacterial inner membrane. Mutagenesis experiments of bases encoding for some of the first 24 residues of VH indicated that the presence of particular basic aminoacids in VH are responsible of the association of the scFv with the bacterial inner membrane, impeding its translocation to the bacterial periplasm. Due to this unexpected situation, the protein can be isolated at a 70% purity by a process that includes the following steps: (a) separation of insoluble and soluble cell material by ultrasound and centrifugation, (b) wash at low urea molarity (2 M) and (c) solubilization at high urea molarity (6 M) (Figure 1). Starting from the solubilized material, the protein can be renatured with extensive dialysis against PBS, pH 8.0, at low temperature. The dialyzed material is then centrifuged, and the soluble phase with the renatured scFv is used for further study.

Procedure (b).- Recognition of the immobilized antigen (EIA polystyrene plates) by the scFv.-

The *E. coli* scFv protein, immunoidentified by Western blot, was submitted to the aforementioned renaturing process and assayed for its ability to recognize the HBsAg antigen, coated onto EIA plates. Briefly, polystyrene plates (Costar) are coated with a pure preparation of the recombinant HBsAg at 5 ug/ml, in carbonate-bicarbonate solution, pH 9.6, overnight at 4°C. The plates are blocked with PBS, pH 7.2 containing 2% skim milk, for 2 hours at 37°C. The different samples are added: (a) renatured scFv, diluted from 1:2 to 1:32 in PBS-1% skim milk, (b) Fab, obtained by enzymatic digestion of MAb CB-Hep.1, at different concentrations, (c) anti-carcinoembryonic antigen scFv as negative control (Ayala et al. 1992. BioTechniques 13:790-799), at 1 ug/ml. The samples are incubated for two hours at room temperature, followed by rabbit antibodies specific for the CB-Hep.1 Fab, at a concentration of 4 ug/ml in PBS-1% skim milk. After 1 hour at 37°C, a Protein A-horseradish peroxidase diluted 1:3000 in PBS-1% skim milk is added, and the final development of the reaction in made with OPD-hydrogen peroxide. Absorbance is read at 492 nm in an automatic plate reader (Multiscan MC340, Flow Laboratories). Extensive plate washing (10 times) was carried out after each step.

Table III

| Recombinant HBsAg recognition by the scFv in ELISA. | |
|---|---|
| Samples | Absorbance at 492 nm |
| Fab of MAb CB-Hep.1 (1ug/ml)<br>anti-HBsAg scFv (renatured)<br>anti-HBsAg scFv (renatured/purified)<br>anti-CEA scFv (periplasm)<br>Periplasm of MM294 (recombinant)<br>Periplasm of MM294(non recombinant) | 0.95<br>1.54<br>0.68<br>0.014<br>0.09<br>0.003 |
| Note: Results in Table show that the renatured scFv recognizes specifically the recombinant HBsAg. | |

Procedure (c). Purification of the scFv using immobilized metal ion affinity chromatography (IMAC).-

The scFv obtained as described in Procedure (a), was dialyzed against O.1 M sodium phosphate, 0.5 M NaCl, pH 8.0 (coupling solution), and applied to a Sepharose-IDA-Ni$^{+2}$ for purification, using the 6-histidine domain present in the protein. It has been reported that domains of such characteristics confer proteins a very high affinity for the aforementioned type of chromatographic support, rendering a simple and reproducible purification procedure (Skerra et. al. 1991. BioTechnology 9:273-278; J. Porath. 1992. Protein Expression and Purification 3: 263-281).

Once the coupling is made, the gel is first washed with 10 times its volume with the coupling solution, but adjusted to pH 6.3, and followed by a similar wash with the solution adjusted to pH 5.0 (for the elution of *E. coli* contaminant proteins). The elution of the scFv proceeds at pH 4.0 with the same solution.

Results on 15% SDS-polyacrylamide gel electrophoresis show a purity level of approximately 90%. The relevant fraction is immediately neutralized with Tris base. The test in the ELISA for specific recognition of the antigen indicated that the eluted scFv maintains its activity (Table III).

Procedure (d). Ability of the scFv to bind the antigen in solution.-

The optimal binding concentrations (saturation) of the purified scFv and Fab (obtained by digestion from MAb CB-Hep.1 and purified) to the recombinant antigen coated to polystyrene plates were determined, as explained before. These were 37 ug/ml and 1 ug/ml, respectively. Each type of antibody fragment at its optimal binding concentration was incubated with the antigen at different molar ratios (for the scFv starting with 1:3.5 and for the Fab with 1:10) in solution for two hours at room temperature. After this time, the mixtures were independently added on the antigen coated plates. The ELISA procedure was developed as described previously. The % of inhibition of the binding of antibody fragment to coated antigen was calculated from the absorbance values (being this percent an index of the capacity of the antibody fragment to bind the antigen in solution).

Table IV

| Inhibition of the binding of the anti-HBsAg scFv to the coated antigen, after incubation with the antigen in solution. | | | | |
|---|---|---|---|---|
| Samples | molar ratio (fragment:antigen) | | | |
| Fab of MAb CB-Hep.1 (1 ug/ml) % inhibition | 1:10<br>96 | 1:5<br>85.9 | 1:2.5<br>65.7 | 1:0<br>0 |
| anti-HBsAg scFv (37 ug/ml) % inhibition | 1:3.5<br>95.1 | 1:0.7<br>76.4 | 1:0.14<br>12.8 | 1:0<br>0 |

Example 4.-

Assay of the scFv for the Immunopurification of the Recombinant HBsAg.

Procedure (a). Immobilization of the anti-HBsAg scFv to Sepharose CL-4B. Studies of binding and elution with the pure antigen.-

For the immobilization of the scFv to cyanogen bromide-activated Sepharose, the purified fragment was dialyzed against a carbonate-bicarbonate buffered solution containing sodium chloride, pH 8.6. The scFv solution, at a concentration of 0.3 mg/ml, was rapidly added to the gel, previously hydrated and washed with carbonate-bicarbonate/sodium chloride buffered solution. The incubation proceeded overnight at room temperature. After this time, the uncoupled fragment was eliminated. A gel without ligand was prepared under the same procedures, as negative control. For the binding-elution assays, a saturating amount of recombinant HBsAg was applied in a solution containing Tris HCl 20 mM pH 7.8 with NaCl. After washing, the antigen was eluted using 3 M potassium thiocyanate in Tris HCl 20 mM pH 7.8. The results shown in Table V indicate that the immobilized scFv has the capacity to bind and elute the recombinant HBsAg.

Table V

| Binding and elution of the recombinant HBsAg by the scFv immobilized on Sepharose CL-4B. | |
|---|---|
| | Amount of eluted HBsAg (mg) |
| Sepharose-scFv | 0.126 |
| Sepharose-no ligand | 0.012 |

Procedure (b) Use of the recombinant scFv fragment for the immunopurification of Hepatitis B surface antigen (HBsAg) from a crude extract.-

The recombinant purified scFv fragment was coupled to Sepharose CL-4B gel, activated with cyanogen bromide, as recommended by the suppliers (Pharmacia, Sweden). The coupling ligand density was about 5 mg of protein per ml of activated gel. As a control of the experiment the monoclonal antibody CB-Hep.1 was coupled to the same matrix using the same protocol. Gels were packed on a 1.2 ml column (0.7 cm diameter) at a linear flow rate of 50 cm/h.

The experiments were performed loading to the column a semi-purified HBsAg preparation obtained from a genetically engineered transformed *Pichia pastoris* yeast, were HBsAg was obtained intracellularly and was extracted by disruption of the cells and further acid precipitation and celite adsorption (European Patent Application No. 480 525). This preparation contains 15-20% pure HBsAg. As a control of this experiment, highly purified HBsAg was used (> 95% pure).

In all the chromatographic runs, the volume of starting material containing 2 mg of HBsAg was applied diluted with 20 mM Tris-HCl, 1 M NaCl, 3 mM EDTA, pH 7.2, at a linear flow rate of 25 cm/h. After this step, the column was washed pumping 5 column volumes with the same buffer at 50 cm/h. The elution step was done using the same buffer + KSCN 3 M. The runs were monitored by absorbance at 280 nm at the column outlet. Results demonstrated that the coupled scFv did recognize the surface antigen with a high specificity. The eluted HBsAg showed a purity higher than 90% by SDS-PAGE, a performance as good as that as obtained with the complete monoclonal antibody. Furthermore the adsorption capacity was as high as with the CB-Hep.1. (See Table VI.)

Table VI

| Results from the inmunopurification of the HBsAg experiments using Seph.CL-4B-scFv and Seph.CL-4B-CB-Hep.1. | | | | |
|---|---|---|---|---|
| Expt. | Ligand | Initial Material | mg HBsAg eluted/ml gel | Eluate purity* |
| 1 | scFv | HBsAg, pure | 0.54 | >90% |
| 2 | scFv | HBsAg, pure | 0.34 | >90% |
| 3 | scFv | HBsAg, pure | 0.51 | >90% |
| 4 | scFv | Crude, semipurif. | 0.45 | >90% |
| 5 | scFv | Crude, semipurif. | 0.26 | >90% |
| 6 | scFv | Crude, semipurif. | 0.42 | >90% |
| 7 | CB-Hep.1 | Crude, semipurif. | 0.52 | >90% |
| 8 | CB-Hep.1 | Crude, semipurif. | 0.57 | >90% |
| 9 | CB-Hep.1 | Crude, semipurif. | 0.37 | >90% |

\* Purity estimated by SDS-PAGE.

Example 5.-

Expression in *E. coli* and purification of an insoluble fusion protein composed by the first 24 aminoacids of the heavy chain variable region of MAb CB-Hep.1 and the *Treponema pallidum* membrane antigen.-

The *Treponema pallidum* membrane antigen (TmpA) was cloned in *E. coli* using PCR and samples of a patient. The TmpA was expressed as a soluble cytoplasmic protein at levels close to 30% of the total bacterial protein. The use of different chromatographic methods and its combinations (size exclusion in gels, ionic exchange) renders purities not greater than 60% by SDS-PAGE. Most important contaminants are bacterial soluble cytoplasmic proteins.

To eliminate such contaminants and facilitate the purification process of the referred antigen, the protein was "insolubilized" by a fusion in its amino-terminus with the first 24 aminoacids of the heavy chain variable region of MAb CB-Hep.1. For this, a fusion gene was constructed using the TmpA base sequence, cloned by PCR, and a synthetic adaptor encoding for the aforementioned 24 residues, added at its 5' end, and inserted in the expression vector pPACIB.1.

The new fusion protein was expressed in *E. coli* W3110 at levels higher than 10% of the total bacterial protein, being now insoluble. It was shown by Western blot with patient sera that the new protein was recognized as containing the TmpA antigen. For the purification of the new fusion protein, the insoluble fraction from ultrasound disrupted recombinant bacteria was treated with urea and guanidium chloride, and loaded in Sepharose-IDA-Cu$^{+2}$ gels (immobilized metal affinity chromatography), and the fusion protein selectively eluted with a final purity level greater than 90% by SDS-PAGE.

Legends to figure 1. 15% Sodium dodecyl sulphate polyacrylamide gel electrophoresis. Expression of the anti-HBsAg scFv in strain M294. Recombinant proteins extraction process.

Lane 1:     Total protein in recombinant MM294
Lane 2:     Supernatant after incubation of bacterial pellet with PBS-Lyzozyme
Lane 3:     Pellet from lane 2
Lane 4:     Supernatant after ultrasound disrupture of pellet from lane 3
Lane 5:     Disrupture pellet
Lane 6:     Supernatant of washed pellet from lane 5 with PBS-2M Urea
Lane 7:     Pellet after washing with PBS-2M Urea
Lane 8:     Supernatant after solubilization with 6M Urea
Lane 9:     Pellet after solubilization
NOTE:     Arrow indicates the position of the 32-33 Kda scFv.

Table VI: <u>Nucleotide and amino acid sequences of scFv</u>

```
gag gtg aag ctg gat gaa act gga gga ggc ttg gtg caa cct
 E   V   K   L   D   E   T   G   G   G   L   V   Q   P


ggg agg ccc atg aaa ctc tcc tgt gtt gcc tct gga ttc act
 G   R   P   M   K   L   S   C   V   A   S   G   F   T


ttt agt gac ttc tgg atg aac tgg gtc cgc cag tct ccg gag
 F   S   E   F   W   M   N   W   V   R   Q   S   P   E
            ------VH.CDR1------


aaa gga ctg gag tgg gta gca caa atc aga gac aaa cct gat
 K   G   L   E   W   V   A   Q   I   R   D   K   P   D
                            ------------------------------


aat tat gca ata tat tat tca gaa tct gtg aaa ggc aga ttc
 N   Y   A   I   Y   Y   S   E   S   V   K   G   R   F
----VH.CDR2----------------------------------------


acc atc tca aga gat gat tcc aga agt agt gtc ttc ctg caa
 T   I   S   R   D   D   S   R   S   S   V   F   L   Q


atg aac agt tta aga cct gaa gat acg ggt atc tat tac tgt
 M   N   S   L   R   P   E   D   T   G   I   Y   Y   C


acg gcc ggt ttt gac tat tgg ggc caa ggc acc act ctc aca
 T   A   G   F   D   Y   W   G   Q   G   T   T   L   T
            ----VH.CDR3----


gtc tcc tca gag ggt aaa tcc tca gga tcc ggc tcc gaa tcc
 V   S   S   E   G   K   S   S   G   S   G   S   E   S
                ----------------------------Linker--------


aaa gtc gac gac att gtg atg tca cag tct cca tcc tcc cta
 K   V   D   D   I   V   M   S   Q   S   P   S   S   L
----------


gct gtg tca gtt gga gag aag gtt gct ttg agc tgc aag tcc
 A   V   S   V   G   E   K   V   A   L   S   C   K   S
                                            -------


agt cag agt ctt tta tat ctt aac aat cac aag aac tac ttg
 S   Q   S   L   L   Y   L   N   N   H   K   N   Y   L
--------------------VL.CDR1-------------------------


gcc tgg ttc cag cag aaa cct ggg cag tct cct aaa ctg ctg
 A   W   F   Q   Q   K   P   G   Q   S   P   K   L   L
---
```

```
att tac tgg gca tcc act agg gat tct ggg gtc cct gat cgc
 I   Y   W   A   S   T   R   D   S   G   V   P   D   R
         -----------VL.CDR2-----------

ttc aca ggc agt gga tct ggg aca gat ttc act ctc atg atc
 F   T   G   S   G   S   G   T   D   F   T   L   M   I

agc agt gtg aag gct gaa gac ctg gca gtt tat tac tgt caa
 S   S   V   K   A   E   D   L   A   V   Y   Y   C   Q
                                                     ---

caa tat tat aat tat ccg tac acg ttc gga ggg ggg acc aag
 Q   Y   Y   N   Y   P   Y   T   F   G   G   G   T   K
         -----------VL.CDR3---------------

ctg gaa ata aaa
 L   E   I   K
```

REFERENCES:

1. Avrameas, S. and T. Ternynck. 1969. The crosslinking of proteins with glutaraldhyde and its uses for the preparation of immunoadsorbents. Immunochemistry 6:53-66

2. Ayala, M., M. Dueñas, A. Santos, J. Vázquez, A. Menéndez, A. Silva and J.V. Gavilondo. 1992. Bacterial single-chain antibody fragment, specific for carcinoembryonic antigen. BioTechniques 13:790-799.

3. Berry, M. J. et. al. 1991. Immobilization of Fv antibody fragments on porous silica and their utility in affinity chromatography. Journal of Chromatography, 587:161-169.

4. Berry, M.J. and J.J. Pierce. 1993. Stability of immunoadsorbents comprising antibody fragments. Comparison of Fv fragments and single-chain Fv fragments. J. Chromatography 629:161-168.

5. Bethell, G.S., J.S. Ayers, W.S. Hancock, and M.T.W. Hearn. 1979. A novel method of activation of cross-linked agarose with 1,1'-carbonyl-diimidazole which give a matrix for affinity chromatography devoid of additional charge groups. J.Biol.Chem. 254:2572-2574

6. Campbell, A. (Edit). 1984. Monoclonal Antibody Technology. Vol.13, Laboratory Techniques in Biochemistry and Molecular Biology, Elsevier Pub. Co.

7. Chase, H.A. 1984. Affinity separations utilising immovilized monoclonal antibody: A new tool for the biochemical engineer. Chem.Eng.Sci. 39:1099-1125.

8. Chaudhary, V.K., J.K. Batra, M.G. Gallo, M.C. Willingham, D.J. Fitzgerald, I. Pastan. 1990. A rapid method of cloning functional variable-region antibody genes in E. coli single chain immunotoxins. Proc. Natl. Acad. Sci. USA 87: 1066-1070.

9. Fontirrochi, G., M. Dueñas, M. E. Fernández de Cossío, P. Fuentes, M. Pérez, D. Mainet, M. Ayala, J.V. Gavilondo, C. Duarte. 1993. A mouse hybridoma cell line secreting IgG And IgM antibodies with specifity for Hepatitis B virus surface antigen. Biotecnología Aplicada 10, 1:24-30.

10. Gavilondo J.V., M.J. Coloma, J. Vazquez, M. Ayala, A. Macías, K.E. Fry and J.W. Larrick. 1990. Specific Amplification of Rearranged Immunoglobulin Variable Region Genes from Mouse Hybridoma Cells. Hybridoma 9:407-417.

11. Gavilondo, J.V., B. Balint, M. Ayala, J. Vázquez, M.E. Fernández-de-Cossío, J.W. Larrick. 1993. pPACIB1: a vector for bacterial expression of scFv antibody fragments. Proceedings of the IV Annual Conference on Antibody Engineering. IBC Conferences Inc. Coronado, CA. December 8-10, 1993. Abstract.

12. Gough, N.M. 1988. Rapid Cloning and quantitative preparation of cytoplasmic RNA from small number of cells. Anal.Biochem. 173:93-95.

13. Handa-Corrigan, A. 1988. Large-scale hybridoma in vitro culture. Bio/Technology 6:784-786

14. Porath, J. 1992. Immobilized Metal Ion Affinity Chromatography. Protein Expression and Purification 3:263-281.

15. Kabat, E.A., T.T. Wu, H.M. Peery, K.S. Gottesman, C. Foeller 1991. Sequencesces of proteins of immunological interest. Vol. 11, 5th. Edition. U.S. Dept. Health and Human Services. NIH Publication No. 91-3242.

16. Mattiasson Bo. 1988. Affinity immobilization. Methods in Enzymology, Vol.137: 647-656.

17. Oste, C. 1988. Polymerase chain reaction. BioTechniques 6:162-167.

18. Office of Biologics Research and Review, Center for Drugs and Biologics, FDA. Points to Consider in the Manufacture and Testing of Monoclonal Antibody Products for Human Use. Memorandum, 1987.

19. Office of Biologics Research and Review, Center for Drugs and Biologics, FDA. Points to Consider in the Production and Testing of New Drugs and Bilogicals Produced by Recombinant DNA Technology. Draft, 1985.

20. Plückthun, A. 1991. Antibody engineering: advances from the use of *E. coli* expression systems. Bio/Technology 9: 545-551.

21. Skerra A., I. Pfizinger and A. Pluckthun. 1991. The functional expression of antibody Fv fragments in E.coli: Improved vectors and a generally applicable purification technique. Bio/Technology 9:273-278.

22. Spitznagel T.M. and D.S. Clark. 1993. Surface-Density and Orientation Effects on Immovilized Antibodies and Antibody Fragments. Bio/Technology 11:825-829.

23. Sada, E., S. Katoh, K. Sukai, M. Tohma, and A. Kondo. 1986. Adsoption equilibrium in immunoaffinity chromatogarphy with policlonal and monoclonal antibodies. Biotechnol.Bioeng. 28:1497-1502.

24. Whitlow, M. and D. Filpula. 1991. Single-chain Fv proteins and their fusion proteins. In: METHODS: A Companion to Methods in Enzymology. Vol 2. No.2. pp 97-105.

EP 0 686 696 A1

SEQUENCE LISTING

SEQ.ID.Nº: 1
SEQUENCE TYPE: Nucleotide with the corresponding Amino acid
SEQUENCE LENGTH: 387
MOLECULE TYPE: Complementary DNA
ORIGINAL SOURCE ORGANISM: Hybridoma cells secreting MAb CB-Hep.1
EXPERIMENTAL SOURCE: *E. coli* transformed with plasmid
pPACIB.1/scFv
FEATURES: From -14 to -1 signal sequence, from 1 to 113 mature
peptide.
PROPERTIES: Gene encoding for variable domain of heavy chain
from AcM CB-Hep.1

```
atgaaatggagctgggttttttctcttaaaaggtgtccagtgt----------leader
  M   K   W   S   W   V   F   L   L   K   G   V   Q   C
-14                                             -1


gaggtgaagctggatgaaactggaggaggcttggtgcaacctgggaggcccatg
  E   V   K   L   D   E   T   G   G   G   L   V   Q   P   G   R   P   M
 +1


aaactctcctgtgttgcctctggattcacttttagt---------------FR1
  K   L   S   C   V   A   S   G   F   T   F   S
                                         30


gacttctggatgaac------------------------------------CDR1
  E   F   W   M   N
              35


tgggtccgccagtctccggagaaaggactggagtgggtagca----------FR2
  W   V   R   Q   S   P   E   K   G   L   E   W   V   A
                                         49


caaatcagagacaaacctgataattatgcaatatattattcagaatctgtgaaa
  Q   I   R   D   K   P   D   N   Y   A   I   Y   Y   S   E   S   V   K
          52  a   b   c


ggc-------------------------------------------------CDR2
  G
 65


agattcaccatctcaagagatgattccagaagtagtgtcttcctgcaaatgaac
  R   F   T   I   S   R   D   D   S   R   S   S   V   F   L   Q   M   N
                                                     82  a


agtttaagacctgaagatacgggtatctattactgtacggcc----------FR3
  S   L   R   P   E   D   T   G   I   Y   Y   C   T   A
  b   c                                     94


ggttttgactat--------------------------------------CDR3
  G   F   D   Y
 95          102


tggggccaaggcaccactctcacagtctcctca------------------FR4
  W   G   Q   G   T   T   L   T   V   S   S
103                              113
```

18

SEQ.ID.Nº: 2
SEQUENCE TYPE: Nucleotide with the corresponding Amino acid
SEQUENCE LENGTH: 399
MOLECULE TYPE: Complementary DNA
ORIGINAL SOURCE ORGANISM: Hybridoma cells secreting MAb CB-Hep.1
EXPERIMENTAL SOURCE: *E. coli* transformed with plasmid
pPACIB.1/scFv
FEATURES: From -20 to -1 signal sequence, from 1 to 107 mature
peptide.
PROPERTIES: Gene coding for variable domain of light chain from
AcM CB-Hep.1

```
atggattcacaggcccaggttcttatgttactgctgctatgggtatctggtacc
 M   D   S   Q   A   Q   V   L   M   L   L   L   W   V   S   G   T
-20

tgtggg---------------------------------------------leader
 C   G
    -1

gacattgtgatgtcacagtctccatcctccctagctgtgtcagttggagagaag
 D   I   V   M   S   Q   S   P   S   S   L   A   V   S   V   G   E   K
+1

gttgctttgagctgc---------------------------------FR1
 V   A   L   S   C
                23

aagtccagtcagagtcttttatatcttaacaatcacaagaactacttggcc--CDR1
 K   S   S   Q   S   L   L   Y   L   N   N   H   K   N   Y   L   A
            27   a   b   c   d   e   f                   34

tggttccagcagaaacctgggcagtctcctaaactgctgatttac-------FR2
 W   F   Q   Q   K   P   G   Q   S   P   K   L   L   I   Y
                                                49

tgggcatccactagggattct-----------------------------CDR2
 W   A   S   T   R   D   S
                56

ggggtccctgatcgcttcacaggcagtggatctgggacagatttcactctcatg
 G   V   P   D   R   F   T   G   S   G   S   G   T   D   F   T   L   M

atcagcagtgtgaaggctgaagacctggcagtttattactgt-----------FR3
 I   S   S   V   K   A   E   D   L   A   V   Y   Y   C
                                                88

caacaatattataattatccgtacacg------------------------CDR3
 Q   Q   Y   Y   N   Y   P   Y   T
                97

ttcggagggggggaccaagctggaaataaaa-----------------------FR4
 F   G   G   G   T   K   L   E   I   K
                                107
```

## Claims

1. Recombinant antibody fragment of the single chain Fv type, characterized by having specificity for the Hepatitis B virus surface antigen and having variable region sequences that essentially comprise the sequences identified with numbers 1 and 2 in the sequence listing.

2. Use of the scFv of claim 1 for immunopurification of HBsAg.

3. Use of the scFv of claim 1 in immunoassays in which the Hepatitis B virus surface antigen is recognized and detected.

4. Use of any antibody fragment, or peptide, natural, synthetic or recombinant, that contains the variable region sequences, or part thereof, defined in claim 1, for immunopurification of recombinant or natural HBsAg.

5. Use of any antibody fragment, or peptide, natural, synthetic or recombinant, that contains the variable region sequences, or part thereof, defined in claim 1, in immunoassays in which recombinant or natural HBsAg is recognized and detected.

6. Use of any antibody fragment, or peptide, natural, synthetic or recombinant, that contains the variable region sequences, or part thereof, defined in claim 1, for insolubilization of heterologous fusion proteins expressed in *E. coli.*

7. A process of preparing an scFv antibody fragment according to claim 1, comprising providing a recombinant nucleic acid containing a nucleotide sequence coding for said scFv antibody fragment and regulatory sequences allowing expression of said scFv antibody fragment in a particular host cell, transforming said particular host cell with said recombinant nucleic acid, growing the transformed host cell and isolating therefrom the scFv antibody fragment produced.

8. The process of claim 7, wherein said host cell is a prokaryotic host cell, e.g. E.coli.

9. The process of claim 7 or 8, wherein the scFv antibody fragment produced is isolated from the insoluble cell fraction.

Figure 1.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | MOLECULES AND CELLS, vol. 4, no. 4, 1994 SEOUL, KOREA, pages 413-417, S. PARK ET AL. 'Expression of soluble and functional single-chain antibody in Escherichia coli.' * the whole document * | 1-9 | C12N15/13 C07K16/08 C07K1/22 G01N33/577 |
| D,Y | BIOTECHNIQUES, vol. 13, no. 5, November 1992 NATICK, MA, USA, pages 790-799, M. AYALA ET AL. 'Bacterial single-chain antibody fragments, specific for carcinoembryonic antigen.' * the whole document * | 1-9 | |
| D,Y | EP-A-0 480 525 (CENTRO DE INGINIERIA GENETICA Y BIOTECNOLOGIA) * example 4 * * claims * | 1-9 | TECHNICAL FIELDS SEARCHED (Int.Cl.6) |
| D,A | BIO/TECHNOLOGY, vol. 11, no. 7, July 1993 NEW YORK, NY, USA, pages 825-829, T. SPITZNAGEL ET AL. 'Surface-density and orientation effects on immobilized antibodies and antibody fragments.' * the whole document * | 1-9 | C12N C07K G01N |

-/--

The present search report has been drawn up for all claims

| Place of search | Date of completion of the search | Examiner |
|---|---|---|
| THE HAGUE | 11 September 1995 | Nooij, F |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,A | BIOTECNOLOGIA APLICADA, vol. 11, no. 2, 1994 LA HABANA, CUBA, pages 175-179, I. ALMAGRO ET AL. 'Purificacion del AgsHB-r por cromatografia de immunoaffinidad. Estudio de los AcMs CB-HEP.1 y CB-HEP.3.' * abstract * | 1-9 | |
| A | EP-A-0 591 548 (SUNTORY LTD.) * the whole document * | 1-9 | |
| A | WO-A-93 24630 (AGEN LTD.) * page 4, line 7 - page 5, line 14 * * example 8 * * claims * | 1-9 | |
| P,X | BIOTECHNIQUES, vol. 18, no. 5, May 1995 NATICK, MA, USA, pages 832-842, M. AYALA ET AL. 'Variable region sequence modulates periplasmic export of a single-chain Fv antibody fragment in Escherichia coli.' * the whole document * | 1-9 | **TECHNICAL FIELDS SEARCHED** (Int.Cl.6) |

The present search report has been drawn up for all claims

| Place of search | Date of completion of the search | Examiner |
|---|---|---|
| THE HAGUE | 11 September 1995 | Nooij, F |